# EUROPEAN PATENT APPLICATION

(11) **EP 4 600 371 A1**
(43) Date of publication of application: **13.08.2025**
(21) Application number: 24156575.3
(22) Date of filing: 08.02.2024
(51) Int. Cl.: C12P 21/00, C07H 11/04, C07K 2/00, C12N 9/10, C12N 9/12

(54) **CELL-FREE GLYCOSYLATION OF PEPTIDES AND PROTEINS**

(71) Applicant: ETH Zurich, 8092 Zurich (CH)
(72) Inventor: LOCHER, Kaspar, 8049 Zürich (CH); RAMIREZ VACA, Ana, 8102 Oberengstringen (CH); ALEXANDER, John, 8102 Oberengstringen (CH); ROSSI, Lorenzo, 8006 Zürich (CH)

(57) **Abstract**

This invention concerns a covalent conjugate between a monosaccharide and/or glycan and the lipid carrier molecule phytol as well as a cell-free *in vitro* method and a cell free *in vitro* system for forming of a glycosylated peptide or polypeptide using said conjugate. A peptide or polypeptide comprising one or more glycosyl-moiety acceptor residues is contacted with a covalent conjugate between a monosaccharide and/or glycan and the lipid carrier molecule phytol in the presence of an oligosaccharyltransferase to form a glycosylated peptide or polypeptide. The invention also concerns a peptide or polypeptide with a glycosyl-moiety acceptor residue having an exogenous *N*-linked glycosylation sequence L(A/V)NYT, as well as an N-glycosylated conjugate of said peptide or polypeptide.

## Description

### Technical domain

The present invention concerns *in vitro* assembly of glycans for glycosylation of peptides and proteins.

### Related art

Glyco-enhanced therapeutics hold great promise as effective treatment for several human diseases. Compared to their unmodified counterparts, glycopeptides display superior pharmacological properties that heavily rely on the presence of high-mannose glycans. Currently, the production of homogenous glycopeptides is restricted by the complexity and diversity of glycans present in living cells and by the challenges of maintaining regio- and stereoselectivity during chemical synthesis or modification.

The posttranslational modification of protein asparagine residues with glycans, termed *N*-linked glycosylation, is ubiquitously observed across the three domains of life, underscoring its physiological significance. N-glycans exhibit notable diversity in both size and sugar composition, thereby facilitating a wide array of distinct biological functions. In eukaryotes, *N*-glycans are assembled onto the lipid dolichol as lipid-linked oligosaccharides (LLOs), a process that takes place on both sides of the endoplasmic reticulum (ER) membrane by the Asparagine-Linked Glycosylation (ALG) enzymes. The enzymatic extension of the glycan ensures strict maintenance of stereochemistry and regioselectivity. The conserved glycan structure transferred to proteins in metazoan and fungi is composed of 14 sugar units comprised of three branches. This glycan is subsequently pruned, modified, and extended again in the ER and Golgi.

High-mannose *N*-glycan, GlcNAc₂Man₉Glc₃, has a defined structure and composition that is conserved across higher eukaryotes and is essential for recognition by the calnexin calreticulin cycle that monitors protein folding prior to export to the secretory pathway. Further modification of the *N*-glycan occurs in the Golgi apparatus, generating a vast array of distinct glycans observed on mature glycoproteins. These glycan modifications are essential for the correct function of secreted and membrane proteins that are involved in the development and function of the nervous system and have a role in cancer and infectious disease. For example, host *N*-glycans are involved during influenza and SARS-CoV-2 viral infections where they are crucial for pathogen adherence or the function and stabilisation of viral proteins. Additionally, there are more than 50 documented congenital disorders of glycosylation in humans that are caused by malfunctioning of the *N*-linked glycosylation pathway.

In addition to the central functions of *N*-linked glycosylation in biology, *N-*linked glycosylation also has important impact on the pharmacokinetics and pharmacodynamics of biologic therapeutics. The activity and function of many glycopeptide- and protein-based therapeutics require specific glycans to elicit the desired response. The bound *N*-linked glycan structure is a particularly important consideration for therapeutic IgG antibodies, which may have divergent proinflammatory or anti-inflammatory activity depending on the structure of bound *N-*linked glycans. Another example are phosphorylated glycans which play an important role in influencing the localisation of lysosomal enzymes essential for proper functioning of degradation pathways. In addition to influencing the effect of protein-based biologics, *N*-linked glycosylation of peptide therapeutics can improve peptide hydrophilicity, circulation time and cellular uptake while reducing aggregation and proteolysis. Peptide bioavailability can also be positively influenced by glycosylation, possibly facilitating the development of orally administered medications.

Thus, the synthesis of defined, homogeneous *N*-glycans has biotechnological applications wherever glycans are needed for the modification of proteins or peptides, which together have annual sales in the hundreds of billions as treatments for cancer, diabetes, obesity, viral and bacterial infections as well as many other diseases.

While the demand on defined glycan-conjugates is steadily growing, the production of defined *N*-linked glycans at scale has been extremely challenging, holding back biotechnological applications as well as the study of processing enzymes of the *N*-glycosylation pathway.

Glycoproteins and glycopeptides are currently produced in four main ways:
(i) incorporation of glycan labelled amino acids during solid-phase peptide synthesis,
(ii) glycoprotein production in engineered eukaryotic or bacterial systems, (iii) exchange of heterogeneous *N*-linked glycans from cell-based expression with the desired glycan using modified endo-β-N-acetylglucosaminidase, and, more recently, (iv) production in defined cell-free protein expression systems with added glycosylation machinery.

However, today none of these techniques allows large-scale generation of homogeneous N-glycan conjugates.

While solid-phase peptide synthesis (SPPS) for peptides smaller than 50 amino acids is well established, peptide glycosylation is less developed and has much room for improvement. At present, SPPS can incorporate amino acids with single sugars attached, but there are no reliable methods to generate sufficient yields of homogeneous attachments made up of larger glycans due to competing side reactions. Cell-based glycoprotein production frequently results in poorly controllable and heterogeneous glycans, which can limit their usefulness and requires strict protocols. A specific challenge in the generation of glycopeptides is the production of high-mannose glycopeptides, because high-mannose glycans represent intermediates of cellular *N*-glycosylation, which cannot be enriched or purified in large amounts from natural sources. Furthermore, current methods of enzymatic glycosylation using modified endo-β-N-acetylglucosaminidase enzymes often result in incomplete glycan occupancy due to the dual transglycosylase and hydrolase activities associated with these enzymes.

While cell-free glycoprotein synthesis shows intriguing possibilities, one of the bottlenecks of current approaches is the transfer eukaryotic *N*-glycans onto the receiving peptide or polypeptide. This is primarily due to the fact that bacterial oligosaccharyltransferases (OSTs), for example PgIB, coded in the Protein GLycosylation (Pgl) cluster of *Campylobacter jejuni,* are used in enzymatic *in vitro* glycosylation of polypeptides. Bacterial OSTs are however extremely inefficient in transferring eukaryotic glycans, which are the most relevant glycans for pharmaceutical applications. These impediments seriously hamper on the development of therapeutic peptides that require well-defined larger glycans, such as high-mannose-containing peptides.

The invention set out to improve on existing approaches for glycopeptides and glycoproteins production.

### Short disclosure of the invention

An aim of the present invention is to provide for a synthesis of glycans for *N*-glycosylation and/or for the synthesis of glycopeptides and/or glycoproteins which overcomes one or more of the shortcomings and limitations of the state of the art.

It is another aim of this invention to render the synthesis of *N*-glycans for glycosylation of glycopeptides and/or glycoproteins more economical.

It is yet another aim of this invention to provide for a reliable *in vitro* synthesis system suitable for producing higher *N*-glycans with improved product homogeneity compared to prior art methods.

It is yet another aim of the invention to improve the synthesis of eukaryotic *N*-glycans, respectively of glycoproteins comprising such eukaryotic *N*-glycans.

It is yet another aim of this invention to provide for an alternative *in vitro* synthesis method for *N*-glycans, glycopeptides and/or glycoproteins.

According to the invention, one or more of these aims are attained by the object of the attached claims, and especially by the independent claims. The dependent claims provide optional embodiments of this invention.

In particular, one or more of these aims are attained by a covalent conjugate between a monosaccharide and/or glycan and a lipid carrier molecule phytol.

In this covalent conjugate the monosaccharide and/or the glycan is conjugated with the phytyl-carrier molecule through a phosphate or a pyrophosphate moiety. This phosphate- or pyrophosphate bridge covalently links the phytol to the monosaccharide or glycan.

The covalent conjugate is adapted to be used in a cell-free *in vitro* system for producing a glycosylated peptide or polypeptide. The terms "polypeptide" and "protein" are used synonymously herein.

Said system comprise an oligosaccharyltransferase (OST), a peptide or polypeptide comprising one or more glycosyl-moiety acceptor residues and one or more of said covalent conjugates.

Phytol, sometimes also referred to as phytosol, is an acyclic hydrogenated diterpene alcohol with 20 carbon atoms of the following structural formula:

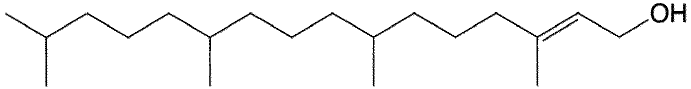

Phytyl phosphate is an acyclic hydrogenated diterpene alcohol phosphorylated at the hydroxyl position of phytol, with 20 carbon atoms of the following structural formula:

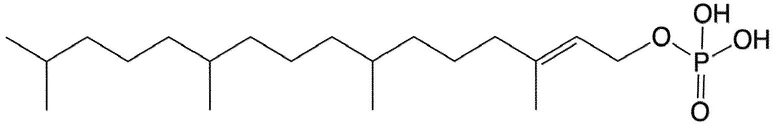

The OST is provided under suitable conditions which enable the enzyme to transfer a glycan from the phytyl-pyrophosphate lipid carrier molecule to a glycosyl-moiety acceptor of the peptide or polypeptide.

The lipid carrier molecule used in this invention is readily commercially available, which drastically simplifies the provision of substrates needed for *N*-linked glycosylation, as no purification or costly synthesis of the lipid carrier is required. The lipid carrier of this invention therefore provides an economical alternative to conventionally used lipid carriers in glycoprotein synthesis.

Advantageously, phytol is not only readily available, but also a non-toxic, non-hazardous compound. Providing phytol as a lipid carrier molecule in the *in vitro* synthesis assay of this invention is therefore environmentally sustainable.

The glycosyl-moiety acceptor residue is preferably an asparagine residue comprised in an N-linked glycosylation sequence of said polypeptide.

The glycan to be transferred may be a mono-antennary, a di-antennary, or a tri-antennary.

The glycan to be transferred may for example be GlcNAc₂, GlcNAc₂Man₁, GlcNAc₂Man₃, GlcNAc₂Man₅, GlcNAc₂Man₆, GlcNAc₂Man₇, GlcNAc₂Man₈, GlcNAc₂Man₉, GlcNAc₂Man₉Glc₁, GlcNAc₂Man₉Glc₂, GlcNAc₂Man₉Glc₃.

OSTs in eukaryotes are generally multi-subunit membrane protein complexes in the endoplasmic reticulum (ER). In bacteria and in some protists OSTs are single-subunit enzymes (ssOST).

In one embodiment the OST in the system is a eukaryotic OST enzyme complex, or a single-subunit eukaryotic OST enzyme.

In one embodiment the OST provided in the system is a STT3A subunit (TbSTT3A) of *Trypanosoma brucei* and/or STT3B subunit of *Trypanosoma brucei* (TbSTT3B), or soluble variants thereof. Whereas TbSTT3A is particularly efficient for transferring *N*-glycans ranging in size from the disaccharide, GlcNAc₂ to GlcNAc₂Man₅, TbSTT3B can also efficiently transfer glycans larger than disaccharides such as GlcNAc₂Man₉ or GlcNAc₂Man₉Glc₃. These enzymes are however also capable of transferring other types of glycans.

The general *N*-linked glycosylation sequence is NX(S/T), where X is any amino acid except proline, and (S/T) signifies that either serine or threonine is provided in the indicated position.

In one embodiment of this invention the *N*-linked glycosylation sequence is SEQ ID No 2
(D/E)ANYT,
where (D/E) signifies that either aspartic acid or glutamic acid is provided in the indicated position. This N-linked glycosylation sequence is the target sequence for TbSTT3A.

In one embodiment of this invention the N-linked glycosylation sequence is SEQ ID No 3
L(A/V)NYT,
where (A/V) signifies that either alanine or valine is provided in the indicated position. This *N*-linked glycosylation sequence is the target sequence for TbSTT3B. This target sequence was identified as part of this invention.

The invention also concerns a peptide or polypeptide comprising an exogenous *N*-linked glycosylation sequence, which is the here disclosed L(A/V)NYT sequence. Said polypeptide may be used as a substrate for an OST, preferably for the STT3B single-subunit eukaryotic OST.

The invention further concerns a nucleic sequence encoding a polypeptide comprising an exogenous L(A/V)NYT sequence, as well as an expression vector comprising sequence.

The invention also comprises a cell containing a nucleic sequence encoding a polypeptide comprising an exogenous L(A/V)NYT sequence.

Different polynucleotides comprising an exogenous L(A/V)NYT sequence may for example be provided in a peptide or polypeptide library. Each peptide or polypeptide member of said library may comprise said exogenous L(A/V)NYT sequence. In one embodiment, each member of the library corresponds to a common parent polypeptide.

This invention also concerns a covalent conjugate between a peptide or polypeptide and a glycan, wherein said peptide or polypeptide comprises one or more exogenous L(A/V)NYT sequence. The glycan is covalently conjugated to said peptide or polypeptide at asparagine (N) residue of said sequence of said *N*-linked glycosylation sequence. The conjugate may be an intermediate of an end product of the glycosylation process of this invention.

For use in the *in vitro* system, the OST is isolated from cells expressing said OST. The OST may for example be a recombinant OST.

While eukaryotic OST enzymes are significantly more efficient and therefore preferred for transferring eukaryotic glycans, in particular higher glycans, it is also possible to use a bacterial ssOST, for example PgIB of *Campylobacter jejuni* in the *in vitro* system. However, it should be noted that bacterial OSTs only very poorly transfer eukaryotic glycans and are therefore better used for the transfer of other types of glycans. It should be noted, that PgIB requires the presence of an acid residue in the -2 position relative to the acceptor asparagine, which is not present in all eukaryotic sequons, including the TbSTT3B sequon, and can therefore not be used with all eukaryotic sequons.

The invention also concerns a cell-free *in vitro* method for producing a glycosylated peptide or polypeptide, in which a peptide or polypeptide containing an *N*-linked glycosylation sequence is glycosylated using an OST and phytol-pyrophosphate-glycan conjugates. Reaction conditions are chosen appropriately, such that the OST transfers the glycan from said covalent conjugates onto the asparagine of said *N*-linked glycosylation sequence, thereby forming a conjugate between said peptide or polypeptide and said glycan. The OST is preferably a eukaryotic OST.

A covalent conjugate between a monosaccharide or a glycan and the lipid carrier may be formed by providing a phosphorylated lipid carrier molecule, such as phosphorylated phytol, also called phytyl-phosphate, providing a nucleotide diphosphate (NDP)-monosaccharide, said NDP may for example be GDP, UDP or ADP, and contacting said NDP-monosaccharide with the phosphorylated lipid carrier molecule phytyl-phosphate under conditions sufficient to covalently link the monosaccharide or glycan to said lipid carrier molecule. Further monosaccharide units may then be added to the monosaccharide-lipid carrier to form a phytyl-glycan conjugate.

Such conditions may for example comprise incubating a reaction mixture comprising an NDP-monosaccharide and/or an NDP-glycan and a phosphorylated lipid carrier molecule of this invention at a temperature ranging from 4°C to 60°C, or 30°C to 50°C, for example at 45°C for a period of time. Said period of time may range from 1h to 24h, or 4h to 16h, or 8h to 12h.

Such conditions may for example comprise the addition of an enzyme for catalysing the transfer of the monosaccharide unit to the phytol carrier molecule. A suitable enzyme is for example an enzyme having dolichylphosphate mannose synthase activity.

For example, GDP-mannose may be incubated with phytyl-P in the presence of an enzyme having dolichylphosphate mannose synthase activity to form phytyl-P-mannose.

As a further example, UDP-N-acetylglucosamine may be incubated with phytyl-P in the presence of an enzyme having phospho-N-acetylglucosamine-transferase activity to form phytyl-PP-N-acetylglucosamine.

Using this method, lipid-linked glycans with either only one phosphate linking the sugar unit to the lipid carrier, or glycans with two phosphates, i.e. with pyrophosphate, linking the sugar unit to the lipid carrier may be generated.

Conjugates containing only one linking phosphate are generally used as sugar donors to elongate a lipid carrier-linked glycan. Conjugates containing a linking pyrophosphate are generally used to transfer a fully assembled glycan onto a protein, This transfer may for example be catalysed by OST, for example the eukaryotic OST TbSTT3A or TbSTT3B.

The monosaccharide-carrier or glycan-carrier conjugate, for example phytyl-P-monosaccharide, phytyl-PP-monosaccharide or phytyl-PP-oligosaccharide, may be used as a starting product or as an intermediate product in the glycosylation of polypeptides. A monosaccharide-carrier-conjugate or an oligosaccharide-carrier conjugate may for example be used to provide additional sugar units to another monosaccharide-carrier conjugate or another oligosaccharide-carrier conjugate. The conjugates may be used to provide different sugar building blocks, comprising one or several sugar units, to assemble a glycan on a carrier molecule of this invention.

It should be noted that eukaryotic OSTs are very inefficient in transferring only one GIcNAc unit. The minimal glycan moiety that these enzymes transfer efficiently is GlcNAc₂.

This invention also concerns said covalent conjugate between a monosaccharide or a glycan and the lipid carrier molecule phytol.

The monosaccharide or the glycan may be conjugated to the phytol through one or more, preferably two, linking phosphate moieties. The phosphate moieties are interposed between the monosaccharide and the lipid carrier, respectively between the glycan and the lipid carrier. An enzyme having phosphoglycosyltransferase activity, for example a homolog of the human phosphoglycosyltransferase DPAGT1, such as ALG7 of *Saccharomyces cerevisiae,* or SaAglH of *Sulfolobus acidocaldarius* (SEQ ID No 14, 15) may for example be used to catalyse the first glycan-lipid carrier link, producing phy-PP-GIcNAc which can be subsequently extended.

Alternatively, a dolichylphosphate mannose synthase of (DPMS), for example PfDPMS of *Pyrococcus furiosus* (SEQ ID No 30, 31), may be used to produce phy-P-Man which is used as the donor substrate by endoplasmic reticulum luminal mannosyltransferases, such as ALG3, ALG9 and ALG12.

The system disclosed herein, respectively its different components, including the lipid-carrier of this invention and/or the modular enzymatic synthesis system, each allow to improve pharmacokinetics and pharmacodynamics of existing peptides and polypeptides. Moreover, the system and its different components permit for the synthesis of novel glycopeptide and glycoprotein therapeutics, especially those that require the addition of larger *N*-glycans which still pose significant challenges for chemical synthesis today.

Additionally, the system and method disclosed here will facilitate the study and diagnosis of congenital disorders of glycosylation by allowing the synthesis of lipid-linked donor and acceptor substrates for assays.

The term "glycan" as used herein refers to a sugar composed of a plurality of monosaccharides. The monosaccharides are attached to one another via glycosidic linkages and may form linear chains or branched chains. The term "glycan" as used herein includes disaccharides, oligosaccharides and polysaccharides. Glycans can be homo- or heteropolymers of monosaccharide residues.

The term "eukaryotic glycan" refers to glycan structures containing a core moiety GlcNAc₂ (GlcNAc-β,1-4-GlcNAc), which is present in all eukaryotic N-glycans and can only be efficiently processed by eukaryotic OSTs.

The term "sequon" refers to a sequence of consecutive amino acids in a peptide or polypeptide which can serve as the attachment site to a glycan, wherein the glycan is linked to the peptide or polypeptide via the nitrogen atom in the side chain of asparagine (N).

The singular "a", "an" and "the" include the plural equivalents unless the context distinctly indicates otherwise.

The terms "comprises" and "contains" mean "includes" in a non-limiting sense.

The terms "e.g.", "for example" and "such as" as used herein indicate specific non-limiting examples that fall under a more general category.

The abbreviations used in this disclosure have the following meanings:

"phy-" stands for phytyl-, "Glc" stands for glucose, "GIcNAc" stands for N-Acetylglucosamine, "Man" stands for mannose, "P" stands for phosphate, "5CF-" stands for 5-carboxyfluorescein-, "LLO" stands for lipid-linked oligosaccharide, "NDP" stands for nucleotide diphosphate, "GDP" stands for guanosine diphosphate, "UDP" stands for uridine diphosphate, "ADP" stands for adenosine diphosphate, and "ATP" stands for adenosine triphosphate.

### Short description of the drawings

Exemplar embodiments of the invention are disclosed in the description and illustrated by the drawings in which:

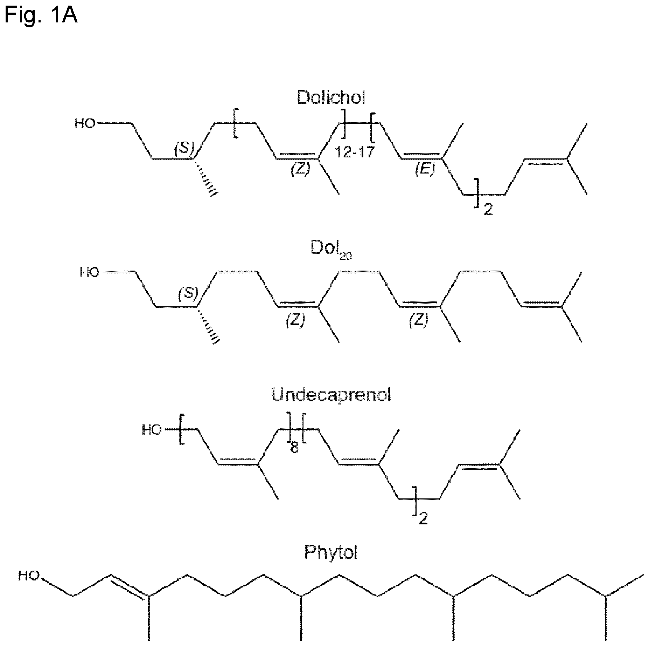

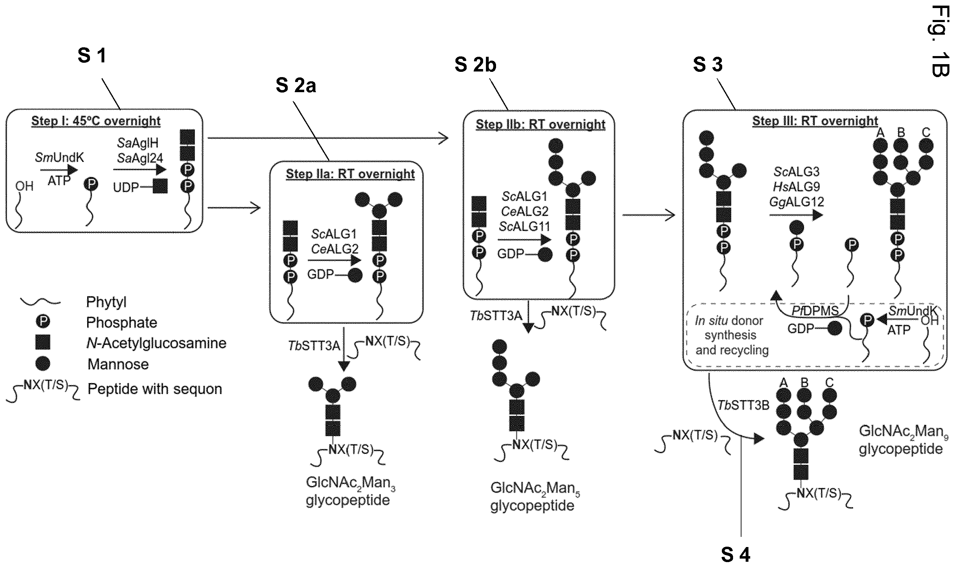

**Figures 1A** **and** **1B** illustrate schematically an example of an *in vitro* high-mannose glycan synthesis and glycopeptide production, wherein
**Figure 1A** depicts different lipid carriers for the synthesis of *N*-glycans, with dolichol, Dol₂₀ and undecaprenol being state- of- the art lipids, and Phytol being a lipid carrier according to this invention; and
Figure 1B is a schematic presentation for the enzymatic synthesis of high mannose *N*-linked glycans on a phytylpyrophosphate (Phy-PP) lipid carrier, in which pre-assembled glycans are transferred to a glycosyl-moiety acceptor residue (N); glycoforms are depicted using the indicated symbol nomenclature;
**Figure 1C** depicts examples of the lipid-linked N-glycan structures using the indicated symbol nomenclature;
**Figures 2A and 2B** showing Tricine gel analysis of fluorescently labelled glycopeptides synthesized using TbSTT3A and TbSTT3B, with the glycopeptides being depicted using the indicated symbol nomenclature, wherein

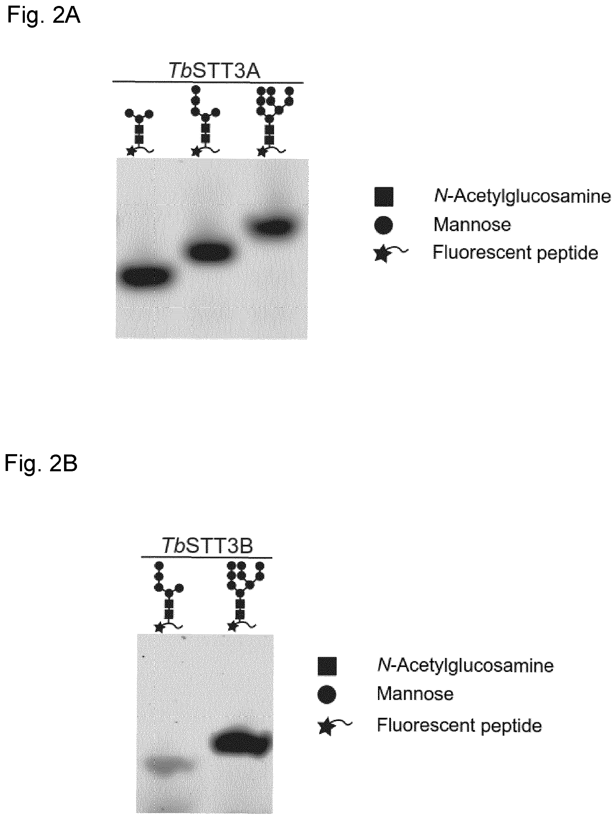

**Figure 2A** shows glycopeptides produced using *T. brucei* STT3A (TbSTT3A) and the glycan donors Phy-PP-GlcNAc₂-Man₃, Phy-PP-GlcNAc₂-Man₅ or Phy-PP-GlcNAc₂-Man₉, linked to a fluorescent peptide comprising the TbSTT3A target sequence; and
**Figure 2B** shows glycopeptides produced using *T. brucei* STT3B (TbSTT3B) and the glycan donors Phy-PP-GlcNAc₂-Man₅ or Phy-PP-GlcNAc₂-Man₉, linked to a fluorescent peptide comprising the TbSTT3B target sequence, wherein the glycopeptides are depicted using the indicated symbol nomenclature;

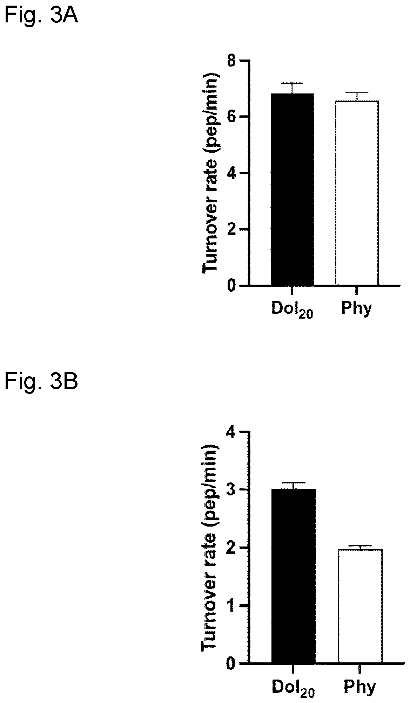

**Figures 3A and 3B** shows the results of Kinetic studies of TbSTT3A and TbSTT3B with phytyl-linked high-mannose glycans (Phy) and Dol₂₀-linked high-mannose glycans (Dol₂₀), with data (n=3) being presented as mean values ± SE; wherein
**Figure 3A** shows TbSTT3A turnover rates for Dol₂₀-PP-GlcNAc₂Man₅ and Phy-PP-GlcNAc₂Man₅; and
**Figure 3B** shows TbSTT3B turnover rates for Dol₂₀-PP-GlcNAc₂Man₉ and Phy-PP-GlcNAc₂Man₉, and

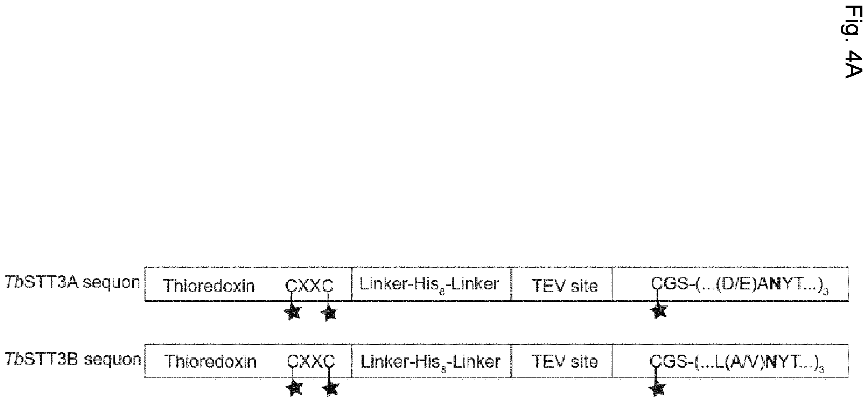

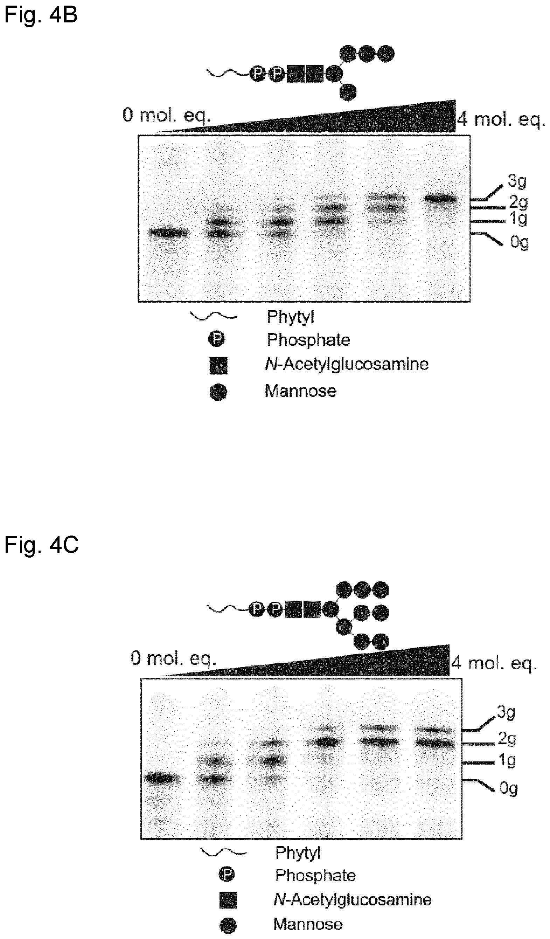

**Figures 4A****,** **4B and 4C** showing design and SDS-PAGE analysis of glycoprotein synthesized according to this invention, with glycoforms in Figures 4A and 4B being depicted using the indicated symbol nomenclature, wherein
**Figure 4A** schematically illustrates Trx-3(TbSTT3A-sequon) (SEQ ID No 6) and a Trx-3(TbSTT3A-sequon) (SEQ ID No 7) construct designs of synthetic proteins with three C-terminal glycosylation sites, the glycosylation sequons are indicated in brackets with the asparagine (N) receiving the glycan being highlighted in bold, the cysteine (C) residues bound to fluorescein are indicated with a star;
**Figure 4B** shows an example of a glycoprotein synthesis according to the method of this invention using TbSTT3A and Phy-PP-GlcNAc₂Man₅, in which fluorescently labelled Trx-3(TbSTT3A-sequon) (SEQ ID No 6) was incubated with 0-4 molar equivalents of Phy-PP-GlcNAc₂Man₅ before the products were separated on an SDS-PAGE gel; and
**Figure 4C** shows an example of a glycoprotein synthesis according to the method of this invention using TbSTT3B and Phy-PP-GlcNAc₂Man₉, in which fluorescently labelled Trx-3(TbSTT3B-sequon) (SEQ ID No 7) was incubated with 0-4 molar equivalents of Phy-PP-GlcNAc₂Man₉ before the products were separated on an SDS-PAGE gel.

### Examples of embodiments of the present invention

### Natural biosynthesis of N-glycans in cells

In living cells, the biosynthesis of the *N*-glycans is initiated on the cytoplasmic face of the ER membrane with the phosphorylation of dolichol (Dol), an isoprene lipid, by the kinase SEC59, followed by the addition of the nucleotide activated sugars, uridine diphosphate N-acetylglucosamine (UDP-GIcNAc) and guanosine diphosphate mannose (GDP-Man). The transfer of an N-acetylglucosamine-1-phosphate (GIcNAc-P) unit to Dol-P is catalysed by the phosphoglucosyltransferase ALG7, yielding Dol-PP-GIcNAc.

The addition of the second GIcNAc unit onto the growing LLO unit is catalysed by the ALG13/14 complex. Subsequently, the cytoplasmic mannosyltransferases ALG1 ALG2 and ALG11 add 5 mannoses to the growing LLO. ALG1 catalyses the transfer of a β1-4 Man unit, which is the acceptor of the following two mannoses (α1-3 and α1-6) transferred by ALG2. ALG11 starts the elongation of a branch A by adding two α1-2 mannoses resulting in resulting DolPP-GlcNAc₂Man₅.

For the biosynthesis of the higher 14-unit N-glycan the DolPP-GlcNAc₂Man₅ is flipped to the luminal side of the of ER membrane for further extension.

In the ER lumen, the mannosyltransferases ALG3 and ALG12 add a Man unit each to the α6 Man previously transferred by ALG2, beginning a B- and a C-branch, which are then both completed with α2 Man additions catalysed by ALG9. First, the B-branch is synthesized by the sequential activity of ALG3 and ALG9. Subsequently, ALG12 catalyses the initiation of the C-branch, which is terminated by ALG9, which adds the second mannose. In the last steps of LLO biosynthesis, the glucosyltransferases ALG6 and ALG8 consecutively add α1-3 glucose unit each to the branch A. Finally, ALG10 ready the *N*-linked glycan for transfer to proteins by adding the terminal α1-2 glucose unit.

Following synthesis of the complete lipid-linked N-glycan, an oligosaccharyltransferase (OST) enzyme catalyses the en bloc transfer of the glycan to an acceptor protein asparagine residue with the core recognition sequon NX(S/T), where N is asparagine, X is any amino acid except proline, S is serine and T is threonine.

### SYNTHESIS PLATFORM FOR LIPID-LINKED EUKARYOTIC GLYCANS, GLYCOPEPTIDES AND GLYCOPROTEINS

### An enzymatic synthesis system

As mentioned above, in eukaryotic cells, glycans are assembled onto the complex lipidic molecule dolichol, which has unfavourable physical chemical properties for *in vitro* synthesis of glycoproteins. Since dolichol cannot be purified in high yields and purity, a cumbersome and expensive chemical synthesis was developed to replace dolichol by a shortened dolichol analogue Dol₂₀. Due to the lack of readily available and low-cost lipid carrier for glycans, *in vitro* glycosylation of polypeptides remains complex, laborious and costly today.

In order to overcome these limitations, the inventors set out to find an inexpensive, commercially available lipid-alcohol precursor which would be suitable as acceptor and donor of phosphate, pyrophosphate and glycosyl-moieties. The lipid carrier should furthermore comprise a lipid scaffold which is sufficiently soluble in aqueous solution to facilitate its use during LLO assembly, as well as in kinetic assays and structural studies.

Of the series of different molecules tested, phytol corresponded to all these requirements. It could be shown that phosphate, pyrophosphate and glycosyl-moieties could be enzymatically added to phytol and that it showed extremely good turn-over rates compared to the conventionally used dolichol analogue Dol₂₀ lipid carrier. Phytol is relatively inexpensive. It can be purchased today at about 1 USD per gram today. Moreover, phytol is readily available from established vendors. It is similar in length to short dolichol analogues which have been shown to be soluble and active with *N*-linked glycosylation enzymes (Fig. 1A).

Phytol was used as a lipid carrier in the *in vitro* synthesis of this study, which used a pipeline of enzymes to transfer and assemble glycans, in particular the eukaryotic GlcNAc₂Man₅ and GlcNAc₂Man₉ *in vitro,* as shown in Figure 1B.

Undecaprenol kinase UndK from *Streptococcus mutans* (SEQ ID No 12, 13) was used to produce phytylphosphate (Phy-P) (Figure 1B, Step I).

In Figures 1B, 2A and B, as well as Figures 4B and 4C, the phytyl-moiety is indicated.

It should be noted that the *in vitro* synthesis system for glycopeptides and glycoproteins of this invention can also use other suitable lipid carrier molecules, such as the conventionally used dolichol and/or its analogues, or analogues of phytol. In this case the phytyl-moiety indicated in the Figures and mentioned in the subsequent paragraphs is replaced by the suitable alternative lipid carrier molecule.

The *in vitro* synthesis system of this invention comprises an enzymatic pipeline for the synthesis of an *N*-glycan.

The suitability of stable enzymes that could modify phy-P into Phy-PP-GlcNAc₂ was assessed for the glycan-elongation steps. As mentioned above, the identified enzymes may also modify other phosphorylated lipid carrier molecules. It was hypothesised that homologs from a thermophilic archaeal species would be better suited in terms of stability and expression than most eukaryotic constructs. Since the species within Crenarchaeota use a dolichylpyrophosphate (Dol-PP) lipid carrier similar to those found in Eukaryotes, the enzymes from the thermophilic crenarchaeal specified *Sulfolobus acidocaldarius* were used in the pipeline. SaAglH (SEQ ID No 14, 15), a homolog of the human phosphoglycosyltransferase DPAGT1 (ALG7 in yeast) and SaAgl24 (SEQ ID No 16, 17), a single subunit homolog of the eukaryotic ALG13/ALG14 complex, were expressed and purified. A one-pot reaction comprising SmUndK (SEQ ID No 12, 13), SaAglH and SaAgl24 was incubated at 45°C overnight. The reaction resulted in GlcNAc₂ linked to phytol through two phosphate groups (phy-PP- GlcNAc₂) as shown in Figure 1B, S1.

For the enzymatic extension of the GlcNAc₂ moiety, previously established techniques for extending glycans attached to a dolichol analogue, as described in Ramírez, A. S. et al.; 2017; Glycobiology 27, 726-733, doi:10.1093/GLYCOB/CWX045, were used, except that the dolichol analogue was replaced by the phytol lipid carrier, the ALG2 homolog from *Caenorhabditis elegans* was used instead of the human homolog and the reaction with ALG1, ALG2 and ALG11 was performed in a one-pot reaction using optimized conditions. A recombinantly expressed and purified mannosyltransferases ALG1 from *Saccharomyces cerevisiae* (ScALG1) (SEQ ID No 18, 19), ALG2 from *Caenorhabditis elegans* (CeALG2) (SEQ ID No 20, 21), and ALG11 from *Saccharomyces cerevisiae* (ScALG11) (SEQ ID No 22, 23) were used to synthesize Phy-PP-GlcNAc₂Man₅. A quantitative conversion to Phy-PP-GlcNAc₂-Man, Phy-PP-GlcNAc₂-Man₃ and Phy-PP-GlcNAc₂-Man₅ was observed in single pot reactions upon incubation of the reaction mixture at room temperature overnight. Phy-PP-GlcNAc₂-Man was formed when only ScALG1 was provided in the reaction mixture. Phy-PP-GlcNAc₂-Man₃ was formed when ScALG1 and CeALG2 were provided in the reaction mixture in the absence of ScALG11 (Figure 1B, S 2a). Phy-PP-GlcNAc₂-Man₅ was formed when ScALG1, CeALG2 and ScALG11 were provided together in the reaction mixture (Figure 1B, S 2b)

A newly synthesised mannose donor may be provided to the enzymatic *in vitro* system. Alternatively, a recycled mannose donor may be provided.

An enzymatic synthesis system of *N*-glycans therefore preferably comprises at least one of SaAglH (SEQ ID No 14, 15) and/or CeALG2 (SEQ ID No 20, 21). The enzymatic synthesis system of N-glycans may further comprise the ALG9 polypeptide of *Homo sapiens* HsALG9 (SEQ ID No 26, 27) or a homolog thereof, the ALG12 polypeptide of *Gallus gallus* GgALG12 (SEQ ID No 28, 29) or a homolog thereof, and/or the ALG3 polypeptide of *Saccharomyces cerevisiae* ScALG3 (SEQ ID No 24, 25) or a homolog thereof. A homolog of the respective enzymes should at least share 40%, 45%, 50%, 55%, 60%, 65%, 70%, 75%, 80%, 85%, 90%, 95%, 96%, 97%, 98%, 99% (or a value between any two of the foregoing values) or greater sequence identity, or at least 50%, 60%, 70%, 80%, 90%, 95%, 96%, 97%, 98%, 99% (or a value between any two of the foregoing values) or greater sequence similarity, as calculated by NCBI BLAST, using default parameters, to the respective aa sequences.

In an optional subsequent step S3 (Figure 1B), phy-PP-GlcNAc₂Man₉ may be synthesised from the intermediate product phytyl-PP-GlcNAc₂Man₅. A recycled phy-P-Man donor substrate may be used for this synthesis.

The synthesis of phy-PP-GlcNAc₂Man₉ or another lipid carrier-GlcNAc₂Man₉ conjugate, may comprise one or more of the enzymatic steps described in the following.

Phy-PP-GlcNAc₂-Man₆ (B branch) was formed when only ScALG3 was provided in the reaction mixture with phy-P-Man and phytyl-PP-GlcNAc₂Man₅.

Phy-PP-GlcNAc₂-Man₆ (C branch) was formed when only GgALG12 was provided in the reaction mixture with phy-P-Man and phytyl-PP-GlcNAc₂Man₅.

Phy-PP-GlcNAc₂-Man₇ (one mannose on B and C branch) was formed when ScALG3 was incubated with phy-P-Man and phytyl-PP-GlcNAc₂Man₅, followed by incubation with GgALG12 (Figure 1B, S3).

Phy-PP-GlcNAc₂-Man₇ (two mannoses on B branch) was formed when ScALG3 and HsALG9 were incubated with phy-P-Man and phytyl-PP-GlcNAc₂Man₅ (Figure 1B, S3).

Phy-PP-GlcNAc₂-Man₇ (two mannoses on C branch) was formed when GgALG12 and HsALG9 were incubated with phy-P-Man and phytyl-PP-GlcNAc₂Man₅ (Figure 1B, S3).

Phy-PP-GlcNAc₂-Man₈ (two mannoses on B branch, one mannose on C branch) was formed when ScALG3 and HsALG9 were incubated with phy-P-Man and phytyl-PP-GlcNAc₂Man₅, followed by denaturation of the latter, for example by boiling the sample, cooling down, and subsequent addition of GgALG12 (Figure 1B, S3).

Phy-PP-GlcNAc₂-Man₉ was formed when ScALG3, GgALG12 and HsALG9 were provided together in the reaction mixture with phy-P-Man and phytyl-PP-GlcNAc₂Man₅ (Figure 1B, S3).

The ratio and identity of sugar residues in the glycans transferred to the peptides were confirmed using mass spectrometry.

This disclosure provides for an enzymatic synthesis system for N-glycans comprising one or more of the selected enzymes mentioned in the previous paragraphs. The enzymatic synthesis system is particularly efficient when combinations of these enzymes are used in one or more of steps S1, S2a, S2b or S3 as described above. The system may comprise an additional step S 4 (Figure 1B) in which the synthesised glycan is transferred onto a peptide or a protein using an OST, for example a eukaryotic ssOST, such as TbSTT3B or TbSTT3A.

The enzymes of this system described herein can be stored over long periods of time without noticeable loss of activity. In addition, it was found, that these enzymes can be re-used multiple times and/or over many days. The enzymatic synthesis system provides therefore robust and reliable glycosylation of peptides and polypeptides. In addition, the system is modular and can therefore be conveniently adapted. Moreover, it provides an environmentally friendly alternative to synthetic glycosylation methods.

Providing selected combinations of these enzymes, or adapting the sequence in which these combinations are provided, allows to control the regio- and stereo-selectivity of glycan synthesis.

Remarkably, the use of these purified enzymes resulted in high reaction yields, outstanding purity and high homogeneity, while offering the possibility to synthesise a high diversity of different glycans compared to previously described methods. In fact, the synthesis reactions described herein routinely achieved close to 100% completion under the tested conditions, thereby avoiding heterogeneity and unwanted side reactions.

The enzymatic synthesis system described herein is particularly adapted for the production of eukaryotic *N*-glycans, including GlcNAc₂Man₅ and GlcNAc₂Man₉.

While phytol may be used as a suitable lipid carrier molecule for monosaccharides and for glycans, the enzymatic synthesis system is not particularly limited to this carrier molecule. Other lipid carriers which are capable of acting as *N-*glycan acceptor or donors may also be used in combination with one or more enzymes of this enzymatic synthesis system.

The modular nature of the enzymatic synthesis system also allows to generate specific intermediate or modified glycans that could incorporate sugars with additional functional moieties or undergo further modification to generate complex *N-*glycans.

Lipid carrier-linked glycans produced using these methods could also be used in endo-β-N-acetylglucosaminidase catalysed reactions where these reactions are successful and glycosylation with an oligosaccharyltransferase is not feasible.

Preferably, an *in vitro* synthesis system of *N*-glycosylated peptides and/or proteins comprises an enzymatic step for the transfer of an *N*-glycan from a lipid carrier molecule, such as phytol, to a peptide or protein. The *N*-glycan-lipid carrier conjugate may be provided to the system. The *N*-glycan-lipid carrier conjugate may be synthesised *in vitro,* for example using one or more of the synthesis steps described above.

Preferably, an enzymatic synthesis system for glycosylation of peptides or proteins contains a eukaryotic single-subunit OST sharing at least 80%, 90%, 95%, 96%, 97%, 98%, 99% (or a value between any two of the foregoing values) sequence identity, or at least 85%, 90%, 95%, 96%, 97%, 98%, 99% (or a value between any two of the foregoing values) sequence similarity, as calculated by NCBI BLAST, using default parameters, to TbSTT3B (SEQ ID No 11).

The ssOST is preferably TbSTT3B (SEQ ID No 10, 11).

### An alternative to the conventional lipid carriers dolichol and dolichol analogues

In the living cell, ER luminal glycosyltransferases which elongate Dol-PP-GlcNAc₂Man₅ use dolichylphosphate (Dol-P)-linked sugars as donor substrates. In vivo, Dol-P-Glc and Dol-P-Man are produced by the enzymes ALG5 and dolichylphosphate mannose synthase (DPMS), respectively.

Neither Dol-P-Man nor Dol-P-Glc are commercially available and are poorly soluble in aqueous solvent, but substrate analogues have been chemically synthesized using complicated, multistep procedures involving shorted analogues of dolichylphosphate.

To better adapt the platform for biotechnological applications, alternative options for these conventionally used enzymes were investigated.

It was found that phy-P-Man could successfully be synthesised in a coupled reaction with UndK, for example the UndK enzyme of S. mutans SmUndK, and the single-subunit homolog of the DPMS from the thermophilic archaeal species, Pyrococcus furiosus (PfDPMS)

It was furthermore found that phy-P-Man can successfully serves as a mannose donor for luminal ALG mannosyltransferase catalysed reactions.

Homolog screening was used to identify the mannosyltransferases ALG3 from *Saccharomyces cerevisiae,* ALG9 from *Homo sapiens,* and ALG12 from *Gallus gallus,* as they all expressed well in suspension cultured HEK293 cells. Purification of the expressed enzymes yielded stable proteins which were highly active *in vitro.*

Using these homologs, phy-P-Man as a donor substrate, as well as a phy-P-Man regeneration system, we demonstrate complete synthesis of phy-PP-GlcNAc₂Man₉ (Figure 1B, S3).

Conveniently, the synthesis of phy-P-Man may also be carried out in the same reaction mix alongside ALG3, ALG9 and ALG12 enzymes used for extension of phy-PP-GlcNAc₂Man₅ to phy-PP-GlcNAc₂Man₉, allowing the continuous re-synthesis of phy-P-Man during extension of the acceptor substrate. No extraction of phy-PP-GlcNAc₂Man₅ from the reaction mixture is therefore required prior to further assembly of Man units to generate phy-PP-GlcNAc₂Man₉, rendering the synthesis of phy-PP-GlcNAc₂Man₉ and higher mannose glycans even more efficient and less time-intensive.

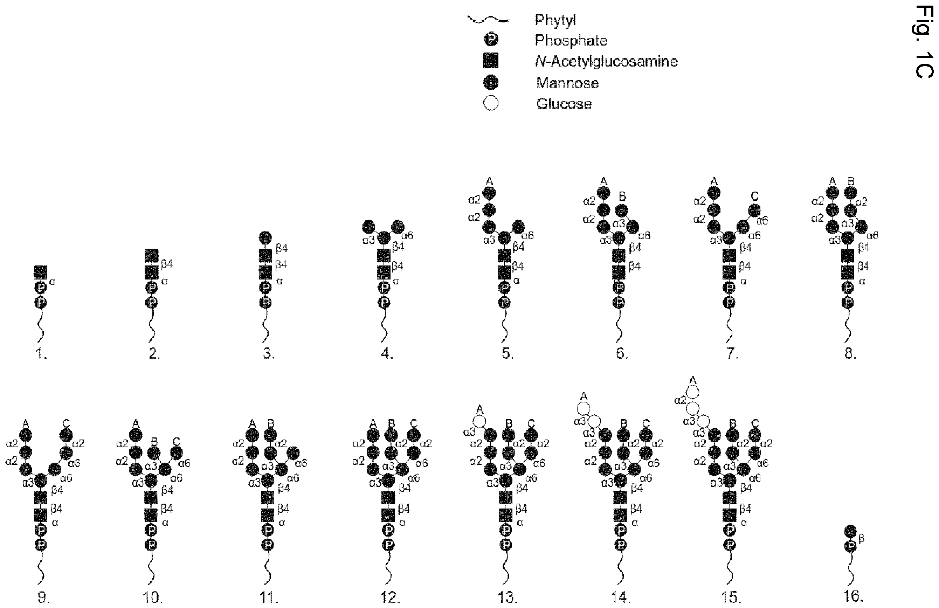

Examples of the lipid-linked N-glycan structures of conjugates according to this invention are provided in Figure 1C, wherein the symbol nomenclature is indicated in the legend. The depicted conjugate each shown a phytol lipid carrier conjugated to a glycan through a pyrophosphate or phosphate linker.

The depicted conjugates are: 1. phy-PP-GIcNAc, 2. phy-PP-GlcNAc₂, 3. phy-PP-GlcNAc₂Man, 4. phy-PP-GlcNAc₂Man₃, 5. phy-PP-GlcNAc₂Man₅, 6. phy-PP-GlcNAc₂Man₆ B-branch, 7. phy-PP-GlcNAc₂Man₆C-branch, 8. phy-PP-GlcNAc₂Man₇ B-branch, 9. phy-PP-GlcNAc₂Man₇C-branch; 10. phy-PP-GlcNAc₂Man₇ B- and C-branches, 11. phy-PP-GlcNAc₂Man₈, 12. phy-PP-GlcNAc₂Man₉, 13. phy-PP-GlcNAc₂Man₉Glc, 14. phy-PP-GlcNAc₂Man₉Glc₂, 15. phy-PP-GlcNAc₂Man₉Glc₃, and 16. phy-P-Man.

The different glycans of this study, respectively the addition of the relevant sugar units, was confirmed by transferring the synthesised glycans onto fluorescently-labelled glycopeptides synthesized using the *Trypanosoma brucei* oligosaccharyltransferases STT3A (TbSTT3A, Figure 2A) and STT3B (TbSTT3B, Figure 2B).

In Figure 2A gel-electrophoresis of labelled glycopeptides produced using TbSTT3A, fluorescent peptide (5CF-GSDANYTYTQ) (SEQ ID No 1) and phy-PP-linked glycans is shown, wherein Peptide -GlcNAc₂-Man₃ is shown in the left lane, Peptide-GlcNAc₂-Man₅ is shown in the middle lane, and peptide-GlcNAc₂-Man₉ in shown in the right lane.

In Figure 2B gel-electrophoresis of labelled glycopeptides produced using TbSTT3A, a 5-Carboxytetramethylrhodamine (TAMRA) fluorescently-labelled peptide (5-TAMRA-YANATS) (SEQ ID No 32), and phy-PP-linked glycans is shown, wherein peptide-GlcNAc2-Man5 is shown in the left lane and peptide-GIcNAc2-Man9 is shown in the right lane.

The curved line in the schematic presentations of the glycopeptides in Figures 2A and 2B indicates the peptide, with the attached fluorophore being depicted as a star. The glycoforms are indicated using the same symbols as for Figure 1B.

The lipid carrier-linked glycans produced according to this invention are suitable for the production of glycopeptides and glycoproteins.

Additionally or alternatively, the lipid carrier-linked glycans of this invention may be used to study ALG pathway enzymes and/or other ER or Golgi-resident enzymes.

### Eukaryotic T. brucei oligosaccharyltransferase single-subunits STT3A and STT3B in vitro

The initial transfer of a complex glycan in protein *N*-glycosylation is catalyzed by the oligosaccharyltransferase (OST) enzyme, which is generally a multi-subunit membrane protein complex in the endoplasmic reticulum of eukaryotes, but a single-subunit enzyme (ssOST) in some protists.

Bacterial OSTs which have previously been used for *in vitro* glycosylation of polypeptides, are not very efficient for transferring eukaryotic *N*-glycans. In a previous study, the inventors have shown, that the use of a subunit of a eukaryotic OST is significantly more efficient than their bacterial ssOST counterparts for transferring eukaryotic *N*-glycans (Ramírez, A. S. et al.; 2017; Glycobiology 27, 726-733, doi:10.1093/GLYCOB/CWX045).

In their previous work, the inventors demonstrated that the eukaryotic oligosaccharyltransferase subunit STT3A of the parasite *Trypanosoma brucei* (TbSTT3A) facilitated high transfer yields of eukaryotic glycans. *T. brucei* expresses a second OST subunit, TbSTT3B. Both *T. brucei* OST subunits were tested independently from each other as part of this study.

In particular, the ability of these OST subunits TbSTT3A and TbSTT3B to use phytol-linked glycans as substrates for transferring *N*-glycans to polypeptides was investigated. It was observed that the turnover rate of glycosylation was almost identical when using phy-PP-GlcNAc₂Man₅ compared to Dolzo-PP-GIcNAczMans, as shown in Figure 3A.

In the case of TbSTT3B, it was observed that the turnover rate when using Phy-PP-GlcNAc₂Man₉ was somewhat slower compared to using Dol₂₀-PP-GlcNAc₂Man₉ as substrate (Figure 3B). However, after an hour of incubation at room temperature the reaction went to completion with both LLO substrates. These results indicate that phytol-based LLOs can be used *in vitro* for generation of glycopeptides with high efficiency.

To test the production of glycoproteins containing multiple glycosylation sites, a fluorescently labelled *E. coli* thioredoxin construct with three C-terminal glycosylation sequons appropriate for processing by TbSTT3A or TbSTT3B, termed Fluorescein-Trx-3(TbSTT3A-sequon) (SEQ ID No 6) and Fluorescein-Trx-3(TbSTT3B-sequon) (SEQ ID No 7), were synthesised. These sequons are schematically depicted in Figure 4A. Both sequons comprise a Thioredoxin region with a CXXC sequence, a Linker region comprising a sequence of eight histidines, and a Tobacco Etch Virus (TEV) protease cleavage sequence region, followed by a C-terminal region comprising the respective *N*-linked glycosylation sequence.

The previously reported *N*-linked glycosylation sequence for the TbSTT3A subunit is SEQ ID No 2:
(D/E)ANYT,
wherein the asparagine (N) is the glycosyl-moiety acceptor residue, and "/" indicating that either one of the shown amino acids may be present.

The sequence was included three times within the C-terminal region of Trx-3(sequon-TbSTT3A) (SEQ ID No 6) comprising the sequence CGS-(...(D/E)ANYT...)3.

The *N*-linked glycosylation sequence for the TbSTT3B subunit was previously unknown and was identified as part of this study. The glycosylation sequence for this subunit is SEQ ID No 3:
L(A/V)NYT,
wherein the asparagine (N) is the glycosyl-moiety acceptor residue, and "/" indicating that either one of the shown amino acids may be present.

The sequence was included three times within the C-terminal region of Trx-3(sequon-TbSTT3B) (SEQ ID No 7) comprising the sequence CGS-(...L(A/V)NYT...)3.

The sequons were designed to accommodate the acceptor substrate preferences of TbSTT3A, i.e. acidic residue in the -2 position, and TbSTT3B, i.e. small aliphatic residue in the -2 position. Both sequons were fluorescently labelled on cystidine (C) residues as indicated by the stars in Figure 4A.

By performing the glycosylation reaction with sub molar quantities of either phy-PP-GlcNAc₂Man₅ for TbSTT3A or phy-PP-GlcNAc₂Man₉ for TbSTT3B the quantitative glycosylation of each sequon was demonstrated, as shown in Figures 4B and 4C. The Figures show SDS-PAGE gel separations of Fluorescin-Trx-3(TbSTT3A-sequon) (SEQ ID No 6) in Figure 4B, and Fluorescin-Trx-3(TbSTT3B-sequon) (SEQ ID No 7) in Figure 4C following their incubation with 0 to 4 molar equivalents of the respective phytyl- glycans as indicated in the Figures.

The results clearly indicate the reactions with TbSTT3A and phy-PP-GlcNAc₂Man₅ show full glycosylation of all three sequons when sufficient LLO is present (Figure 4B). For TbSTT3B and Phy-PP-GlcNAc₂Man₉, a mixture of double and triple glycosylated glycoproteins is produced in presence of 4 molar equivalents of donor substrate (Figure 4C).

This study addressed existing challenges in the biotechnological production of N-glycan containing glycopeptides and glycoproteins by providing a complete and efficient glycosylation platform, which is suitable for glycosylation of peptides and polypeptides with eukaryotic glycans. The platform covers a variety of different aspects, which may be used as part of this platform or independently, in alternative *in vitro* assays.

*In vitro* glycosylation as described herein is compatible with peptides generated via different methods, including solid phase peptide synthesis (SPPS), recombinant technologies, or purification from natural sources.

In one embodiment of this invention a cell-free *in vitro* platform for enzymatic synthesise of glycoproteins is provided, in which the lipid carrier-linked glycans are derived from commercially available starting materials, including the lipid carrier phytol and in which the transfer of the assembled glycan to the glycosyl-moiety acceptor residue of a peptide or polypeptide is catalysed by an eukaryotic OST or a single-subunit OST, such as STT3A and/or STT3B.

It was found that the commercially available and economical compound phytol is a suitable alternative for dolichol, which is difficult to purify at sufficient yields, and dolichol analogues, which are expensive and onerous to synthesise, as a lipid carrier for *N*-glycans.

Moreover, the platform may contain an enzymatic synthesis system comprising enzymes selected from different eukaryotic, bacterial and archaeal species as disclosed herein. Specific combinations and sequences, such as those as provided in the examples, may be chosen to generate glycans of the desired structure and sequence. The enzymatic synthesis system disclosed herein therefore provides a reliable and dynamic tool kit enabling homogenous synthesis of a specific glycan structure. This tool kit further provides the flexibility to synthesise a broad variety of different glycans, permitting for easy customization of glycan production.

Synthesis of glycans as described herein is compatible with a second layer of enzymatic modifications, for example with galactose or sialic acid, and with cell-free protein expression, enabling modular control of glycopeptide and glycoprotein synthesis depending on the enzymes provided.

Different from bacterial OSTs, the *T. brucei* OST subunits STT3A and STT3B are capable of transferring high-mannose glycans efficiently onto *N*-linked glycosylation sequence of polypeptides. It was shown that these subunits not only accept phytyl-*N*-glycans as substrates, but also that they can glycosylate multiple sites in a target polypeptide. The combination of a phytol provided as a lipid carrier molecule for *N*-glycans or monosaccharides with either one or both of the *T. brucei* OST subunits STT3A and/or STT3B therefore provides for an economical and efficient cell-free *in vitro* synthesis of glycoproteins.

This study also identified for the first time the specific N-linked glycosylation sequence targeted by the TbSTT3B subunit. The identification of this sequence enables the production of recombinant peptides and polypeptides comprising one or more copies of this sequence, thereby adapting these peptides and proteins for glycosylation using the TbSTT3B subunit in *in vitro* reactions.

### METHODS

### Expression and purification of SmUndK

A synthetic gene coding for UndK from *Streptococcus mutans* (Uniprot: Q05888) was codon optimized for expression in *E. coli* (GeneArt, Thermo Fisher Scientific) and cloned into a modified pET vector displaying an N-terminal T7 peptide expression tag and a C-terminal His10 purification tag. For protein expression, transformed *E. coli* BL21 (DE3) cells were grown in Luria-Bertani media supplemented with 100 ug/mL ampicillin at 37ºC, 200 rpm, for several hours. Upon reaching OD600 1.0, protein expression was induced by addition of 1 mM isopropyl β-D-1-thiogalactopyranoside (IPTG) and temperature was set to 16ºC for overnight expression. Cells were harvested by centrifugation, flash frozen in liquid nitrogen and stored at -80ºC until further use. For purification, cells were resuspended in lysis buffer (50 mM Tris/HCl pH 7.4, 300 mM NaCl, 5% glycerol, 25 mM imidazole, 0.05 mg/mL DNasel, 1 mM PMSF, complete^{™} EDTA-free Protease Inhibitor Cocktail (Roche)) and lysed by three cycles of sonication (50% amplitude, 3s ON, 3s OFF, 1 min active sonication time). The lysate was solubilized for 1.5h at 4ºC after addition of dodecyl maltoside (DDM, Anatrace) to 0.5% (w/v) and subsequently centrifuged at 45,000 rpm in a Type Ti45 rotor (Beckman-Coulter) for 40 min to remove insoluble debris. The soluble fraction was collected, filtered with a 0.45 um filter (Millipore Sigma) and loaded on a 5 mL Superflow Ni-NTA (Qiagen) column, pre-equilibrated in equilibration buffer (50 mM Tris/HCl pH 7.4, 300 mM NaCl, 5% glycerol, 10 mM MgCl₂, 0.03 % (w/v) DDM, 25 mM imidazole). After loading, the resin was washed with 10 CV wash buffer (50 mM Tris/HCl pH 7.4, 300 mM NaCl, 5% glycerol, 10 mM MgCl₂, 0.03 % (w/v) DDM, 50 mM imidazole), before elution in the same buffer but containing 250 mM imidazole. Purified SmUndK was immediately desalted using PD-10 columns (Cytiva), pre-equilibrated in desalting buffer (50 mM Tris/HCl pH 7.4, 300 mM NaCl, 10 mM MgCl₂, 0.03 % DDM), and concentrated on a 50 kDa molecular weight cut off Amicon centrifugal filter (Merck Millipore). Protein aliquots were flash frozen in liquid nitrogen and stored until at -80ºC further use. Protein purity and oligomeric state were confirmed by SDS-PAGE and size-exclusion chromatography on a Superdex S200 10/300 GL (GE Healthcare) in desalting buffer, respectively.

### Expression and purification of SaAglH and SaAgl24

Genes from *Sulfolobus acidocaldarius* coding for AgIH (Uniprot ID: P39465) and Agl24 (Uniprot: Q4J9C3) were codon optimized for expression in *Escherichia coli* using GeneArt (Thermo Fisher Scientific) and cloned into a modified pET vector using restriction free cloning. SaAglH was expressed with a C-terminal GSS linker, HRV 3C-cleavage site, eYFP and His8 purification tag. SaAgl24 was tagged as for SaAgIH but without eYFP. Both constructs were expressed using *E. coli* BL21 (DE3) cells grown in ZYP-5052 autoinduction media at 37ºC for 4 hours followed by expression overnight at 16ºC. Cells were harvested by centrifugation, flash frozen in liquid nitrogen and stored at -80ºC until needed. Purification of both constructs was carried out with the same purification scheme at 4ºC. Cells were resuspended in lysis buffer (50 mM HEPES, pH 7.5; 500 mM NaCl) in a 1:5 weight ratio and phenylmethylsulfonyl fluoride and DNAsel (Roche) was added to final concentrations of 1 mM and 5 µg mL⁻¹, respectively. Cells were lysed with one pass in a microfluidizer (M-110P, Microfluidics) with a 200 µm ceramic cell equilibrated in lysis buffer and run at 15,000 psi. Cellular debris was removed by centrifugation at 15,000g for 30 minutes. Membranes from the resulting supernatant were then pelleted by centrifugation at 45,000 rpm in a Type 45Ti rotor (Beckman-Coulter) for 1 hour. Membranes were resuspended in lysis buffer, flash frozen in liquid nitrogen and stored at -80ºC.

Isolated membranes were extracted with 1% (w/v) DDM (Anatrace) for 1 hour before insoluble components were pelleted at 40,000 rpm in a Type 45Ti rotor (Beckman-Coulter) for 30 minutes. Imidazole, pH 7.5, was added to the supernatant to 20 mM before loading on a 5 mL Superflow Ni-NTA (Qiagen) column equilibrated in buffer A (20 mM HEPES, pH 7.5; 500 mM NaCl; 0.017 % (w/v) DDM) with 20 mM imidazole added. Resin was washed with 50 mM imidazole in buffer A before protein elution in buffer A with 250 mM imidazole. Fractions containing the protein of interest were pooled and immediately desalted into buffer B (20 mM HEPES, pH 7.5; 150 mM NaCl; 0.017 % (w/v) DDM). Protein was concentrated on a 50 kDa molecular weight cut off Amicon centrifugal filter (Merck Millipore) to 2-3 mg mL⁻¹ and flash frozen in liquid nitrogen.

### Expression and purification of ScALG1, CeALG2 and ScALG11

Expression and purification of ScALG1 residues from 33-349 (Uniprot ID: P16661), and ScALG11 residues from 46-548 (Uniprot ID: P53954) was optimized from previously published work (Ramírez, A. S. et al.; Glycobiology 27, 726-733, doi:10.1093/GLYCOB/CWX045 (2017)). Briefly, truncated genes coding for ScALG1 and ScALG11 proteins were cloned into pQE80 (Qiagen) with an N-terminal His10 tag followed by two copies of the Z-domain, and a TEV cleavage sequence. Expression was carried out in *E. coli* BL21 (DE3) grown in Terrific Broth (TB) media. The expressed ScALG1 protein (SEQ ID NO 35, 36) and the expressed ScALG11 protein (SEQ ID NO 37, 38) comprised a small deletion in their respective N-terminal sequences. Cells were initially grown at 37ºC until an optical density of 1.3-1.5 at 600 nm was reached. The temperature was then reduced to 16ºC, and protein expression was induced with 0.5 mM IPTG for 16 hours. Following harvesting the cells, all steps were performed at 4ºC. Cells were resuspended in lysis buffer (50 mM HEPES, pH 7.5; 150 mM NaCl; 5 mM β-mercaptoethanol, 20 ng ml-1 DNasel (Roche); 1 mM PMSF; EDTA free complete protease inhibitor (Roche) and lysed in a microfluidizer (M-110P, Microfluidics) at 15,000 psi with a 200 µm ceramic cell equilibrated in lysis buffer. Membranes in the cell lysate were then directly extracted with 1% DDM (Anatrace) and 0.1% cholesteryl hemisuccinate (CHS, Anatrace) for 1 hour. Cellular debris was removed by centrifugation at 40,000 rpm in a Type 45Ti rotor (Beckman-Coulter) for 30 minutes. Imidazole was added to the supernatant to 25 mM before loading onto a 5 mL column of Superflow NTA resin (Qiagen) equilibrated in buffer A (20 mM HEPES, pH 7.5; 150 mM NaCl; 5 mM β-mercaptoethanol; 0.03% (w/v) DDM; 0.006% (w/v) CHS) with 25 mM imidazole. The resin was washed with buffer A with 50 mM imidazole before the protein was eluted with buffer A containing 300 mM imidazole. The eluted protein was immediately desalted into buffer A, concentrated on a 50 kDa molecular weight cutoff membrane (Amicon), flash frozen in liquid nitrogen and stored at -80ºC.

The gene from *Caenorhabditis elegans* coding for ALG2 (Uniprot ID: Q19265) was codon optimized for expression in *E. coli* using GeneArt (Thermo Fisher Scientific) and cloned into a modified pET-19b vector with a His10 affinity tag fused to the C terminus. CeALG2 was expressed using *E. coli* BL21-Gold (DE3). Cells were grown in modified Terrific Broth (TB) medium supplemented with 1% glucose (w/v) at 37ºC to an OD600 of 2.7 - 3.0, before expression was induced by the addition of 1 mM IPTG for 1 h at 25°C. Cells were harvested by centrifugation, flash frozen in liquid nitrogen and stored at -80ºC until needed. All following steps were performed at 4 °C. Cells were resuspended in lysis buffer (50 mM HEPES, pH 7.0; 250 mM NaCl; 3 mM β-mercaptoethanol; 10% glycerol; and 0.5 mM PMSF). Cell lysis was performed in a microfluidizer (M-110P, Microfluidics) at 15,000 psi with a 200 µm ceramic cell equilibrated in lysis buffer. Membranes were pelleted by ultracentrifugation at 35,000 rpm in a Type 45Ti rotor (Beckman-Coulter) for 30 minutes, resuspended in lysis buffer, flash frozen in liquid nitrogen and stored at -80ºC.

Membranes were solubilized with 1% DDM (Anatrace) and 0.1% CHS (Anatrace) for 1 hour. Insoluble debris was removed by centrifugation at 35,000 rpm in a Type 45Ti rotor (Beckman-Coulter) for 30 minutes. Imidazole was added to the supernatant to 25 mM before loading onto a 5 mL column of Superflow NTA resin (Qiagen) equilibrated in buffer A (50 mM HEPES, pH 7.0; 250 mM NaCl; 3 mM β-mercaptoethanol; 25 mM imidazole; 0.02% (w/v) DDM; 0.002% (w/v) CHS). The resin was washed with buffer A containing 60 mM imidazole before the protein was eluted with buffer A containing 200 mM imidazole. The eluted protein was immediately desalted into buffer B (25 mM HEPES, pH 7.0; 150 mM NaCl; 3 mM β-mercaptoethanol; 0.02% (w/v) DDM; 0.002% (w/v) CHS), concentrated on a 50 kDa molecular weight cutoff membrane (Amicon), flash frozen in liquid nitrogen and stored at -80ºC.

### Expression and purification of ScALG3, HsALG9 and GgALG12

Gene sequences coding for *Saccharomyces cerevisiae* ALG3 (Uniprot ID: P38179), Isoform 1 of wild-type human ALG9 (Uniprot ID: Q9H6U8) and *Gallus gallus* ALG12 (Uniprot ID: F1P077) were codon optimized for expression in human cells using GeneArt (Thermo Fisher Scientific) and cloned into a modified pUC57 vector using restriction free cloning. ScALG3 was cloned with a C-terminal HRV 3C-cleaveage sequence followed by eYFP and 1D4 sequences. HsALG9 and GgALG12 where cloned with an N-terminal FLAG tag followed by the eYFP sequence and a HRV 3C-cleavage sequence. All three proteins were transiently expressed in suspension HEK293 EBNA cells maintained at 37°C in humidified incubators with supplemental carbon dioxide. Protein expression was induced via transient transfection with branched polyethylenimine. Cells expressing protein for 2 days were collected by centrifugation, washed with PBS and flash frozen before being stored at -80ºC until needed.

Cells were thawed in lysis buffer (150 mM sodium chloride, 50 mM HEPES pH 7.5, 10% (v/v) glycerol) in a 1:5 (wt/vol) ratio. All subsequent steps were either performed on ice or at 4ºC. Prior to lysis by dounce homogenization, 1 mM phenylmethylsulfonyl fluoride, 20 µg/ml DNAse I (Roche) and a 1:100 (v/v) dilution of Protease inhibitor cocktail (Sigma) was added. Membrane extraction was performed for 1 hour in 1% (w/v) n-dodecyl-β-D-maltopyranoside (DDM), (w/v) 0.2% CHS. The lysate was then centrifuged at 40,000 rpm for 30 minutes in a Type-45Ti (Beckman Coulter) rotor to pellet insolubilized membrane. The supernatant was then incubated with either M2 Flag antibody resin (Sigma) or 1D4 antibody resin (made in house) for 1 hour with rotation. The flow-through was discarded and the resin was washed twice with 15 column volumes (CV) wash buffer (150 mM sodium chloride, 20 mM HEPES pH 7.5, 10% (v/v) glycerol, and 0.017 % DDM, 0.0035 % CHS). HRV 3C protease was added to the column and incubated for 1 hour to before the protein of interest was eluted from the column with wash buffer. The protein was concentrated on an Amicon Ultra 50 kDa molecular mass cut-off centrifugal filter (Merck Millipore). Size exclusion chromatography (SEC) was performed using a Superdex 200 increase 10/300 column (GE Healthcare) equilibrated in SEC buffer (150 mM sodium chloride, 20 mM HEPES pH 7.5, 0.017 % DDM, 0.0035 % CHS) and run at 0.5 mL min⁻¹. Fractions containing the protein of interest were concentrated as before and either directly used or flash frozen in liquid nitrogen and stored at -80ºC.

### Expression and purification of TbSTT3A and TbSTT3B

Expression of *Trypanosoma brucei* STT3A (Uniprot: Q57W34) was performed as previously published (Ramírez, A. S. et al.; Glycobiology 27, 525-535, doi:10.1093/GLYCOB/CWX017 (2017)). A synthetic gene encoding *T. brucei* STT3B (Uniprot: Q57W36) was optimized for expression in insect cells and purchased from GeneScript. After cloning it fused to a N-terminal His10-YFP-3C tag into a pOET1 vector, baculovirus production was performed using flashBAC DNA (Oxford Expression Technologies) in *Spodoptera frugiperda* (Sf9) cells following the manufacturer's instructions. For expression, Sf21 cells were infected at a density of 2.0 × 106 cells/mL and incubated for 48 h. Cells were collected by centrifugation at 6500 × g and washed with phosphate-buffered saline. Cell pellets were frozen in liquid nitrogen and stored at -80°C until the time of use.

Purification of *T. brucei* STT3A and STT3B, was performed by thawing cell pellets and resuspending them in lysis buffer (25 mM K₂HPO₄/NaH₂PO₄, pH 7.0; 250 mM NaCl; 10% w/v Glycerol) supplemented with cOmplete^{™}, EDTA-free Protease Inhibitor Cocktail (Roche), followed by lysis with douncer and solubilization with 1% (w/v) DDM, 0.2% (w/v) CHS for two hours at 4°C. After high-speed centrifugation (35,000 rpm, Ti45i rotor, 30 min) the supernatant was loaded onto a Ni/NTA super flow affinity column (Qiagen), washed with the same lysis buffer but containing 50 mM imidazole, 0.035% (w/v) DDM, 0.007% (w/v) CHS and eluted with the same buffer but containing 200 mM imidazole. The protein was desalted into 20 mM HEPES 7.5; 150 mM NaCl; 5% glycerol (v/v) using a HiPrep 26/10 column (GE Healthcare) and incubated with home-produced 3C protease overnight at 4°C (Walker et al. 1994). *T*. *brucei* STT3A and STT3B were further purified by a reverse Ni/NTA step and size exclusion chromatography (Superdex S200 10/300 GL, GE Healthcare) in desalting buffer.

### Expression and purification of PfDPMS

The gene from *Pyrococcus furiosus* coding for DPMS (Uniprot ID: Q8U4M3) was codon optimized for expression in *E. coli* using GeneArt (Thermo Fisher Scientific) and cloned into a modified pET-19b vector with a His10 affinity tag fused to the N-terminus. PfDPMS was expressed using *E. coli* BL21-Gold (DE3). Cells were grown in modified Terrific Broth (TB) medium supplemented with 1% glycerol (w/v) at 37ºC to an OD600 of 2.7 - 3.0, before expression was induced by the addition of 1 mM IPTG for 16 h at 18°C. Cells were harvested by centrifugation, flash frozen in liquid nitrogen and stored at -80ºC until needed. All following steps were performed at 4 °C. Cells were resuspended in lysis buffer (25 mM NaH₂PO4/K₂HPO4, pH 7.0; 150 mM NaCl and 1 mM PMSF). Cell lysis was performed in a microfluidizer (M-110P, Microfluidics) at 15,000 psi with a 200 µm ceramic cell equilibrated in lysis buffer. Membranes were pelleted by ultracentrifugation at 35,000 rpm in a Type 45Ti rotor (Beckman-Coulter) for 30 minutes, resuspended in lysis buffer, flash frozen in liquid nitrogen and stored at - 80ºC.

Membranes were solubilized with 1% DDM (Anatrace) for 1 hour. Insoluble debris was removed by centrifugation at 35,000 rpm in a Type 45Ti rotor (Beckman-Coulter) for 30 minutes. Imidazole was added to the supernatant to 25 mM before loading onto a 5 mL column of Superflow NTA resin (Qiagen) equilibrated in buffer A (50 mM NaH₂PO4/K₂HPO4, pH 7.0; 200 mM NaCl; 0.02% (w/v) DDM). The resin was washed with buffer A containing 50 mM imidazole before the protein was eluted with buffer A containing 200 mM imidazole. The eluted protein was immediately desalted into buffer B (50 mM HEPES, pH 7.0; 200 mM NaCl; 10mM EDTA; 10 mM EGTA and 0.02% (w/v) DDM), concentrated on a 50 kDa molecular weight cutoff membrane (Amicon), flash frozen in liquid nitrogen and stored at -80ºC.

### Synthesis of the mannose donor for ER-luminal ALG mannosyltransferases

Phytylphosphate-mannose was synthesised as follows: 100 uM of 1%DDM-solubilized phytol was incubated with 2.5 uM UndK, 500 uM ATP, 1 uM DPMS and 100 uM GDP-Man overnight at 45°C in donor synthesis reaction buffer (50 mM Tris/HCl pH 7.4, 200 mM NaCl, 10 mM MgCl₂, 0.03 % DDM). After completion, the reaction mix was heated up to 98°C to possibly denature the enzymes and spun down to remove protein aggregates. The supernatant containing phytylphosphate-mannose was then directly used to provide the donor in reactions with ER-luminal mannosyltransferases.

### Synthesis and purification of phytyl-pyrophosphate-linked eukaryotic N-glycans

Phytol (Sigma Aldrich, W502200) was solubilised with sonication in buffer (20 mM HEPES, pH 7.5; 150 mM NaCl; 1% DDM (w/v)) to form an opaque 10 mM stock suspension which was used for the following steps. Phy-PP-GlcNAc₂ was synthesised with 300 µM phytol, a 5-fold molar excess of UDP-GIcNAc (Sigma), and a 20-fold molar excess of ATP. SmUndK, SaAgIH, and SaAgl24 were included in 50:1, 25:1 and 50:1 substrate:enzyme ratios, respectively. Reactions were carried out in buffer (20 mM HEPES, pH 7.5; 150 mM NaCl; 10 mM MgCl₂; 0.017 % DDM) and incubated at 45ºC overnight. Products were stored at -20ºC until needed.

Synthesis of phytyl-PP-GlcNAc₂Man₃ was performed at RT overnight with 150-200 µM phytyl-PP-GlcNAc₂, a 5-fold molar excess of GDP-Man (Sigma), ScALG1 and CeALG2 included at a 100:1 and 20:1, respective substrate to enzyme molar ratios. Synthesis of phytyl-PP-GlcNAc₂Man₅ was performed as for phytyl-PP-GlcNAc₂Man₃ except ScALG11 was included in a 100:1 molar substrate to enzyme ratio and an 8-fold excess of GDP-Man was used. Reactions were performed in buffer (20 mM HEPES, pH 7.5; 150 mM NaCl; 10 mM MgCl₂ 0.017 % DDM; 0.034% CHS; 5 mM β-mercaptoethanol).

Phytyl-PP-GlcNAc₂Man₉ production was carried out with 100 µM phytyl-PP-GlcNAc₂Man₅, a 10-fold molar excess of GDP-Man, ScALG3, HsALG9, and GgALG12 all with a 100:1 molar substrate to enzyme ratio. Synthesis of the donor substate was concomitantly produced in the same reaction mix by the addition of 50 µM phytol, 1 mM ATP, ~1 µM PfDPMS and 1-2 µM SmUndK. The reaction was performed at RT overnight in buffer (20 mM HEPES, pH 7.5; 150 mM NaCl; 0.017 % DDM; 0.0034 % CHS).

After overnight incubation the reaction was heated to 95ºC for 10 minutes, the reaction was centrifuged for 5 minutes at ~16,000g and the supernatant was collected to remove the denatured enzymes. The substrates were then purified as previously published (REF 64).

### In vitro glycopeptide synthesis

Glycopeptide synthesis was carried out overnight at 30ºC with 10 µM fluorescent peptide, an excess of phytyl-PP-linked oligosaccharide, and 250 nM TbSTT3A or TbSTT3B. For TbSTT3A and TbSTT3B reactions the 5CF-GSDANYTYTQ acceptor peptide (SEQ ID No 1) and TAMRA-YANATS acceptor peptide (SEQ ID No 32) were used respectively. The reactions were performed in 150 mM NaCl, 20 mM HEPES, pH 7.5, 10 mM MnCl₂, 0.035% DDM, and 0.007% CHS. Following the reactions, the samples were diluted in Laemmli buffer, separated using Tricine gel electrophoresis, and visualised on a fluorescence scanner.

### Kinetic assays with ssOST enzymes

*In vitro* glycosylation reactions were performed by mixing 50 nM purified TbSTT3A or 100 nM TbSTT3B protein, 15µM Phy-LLO or Dol₂₀-LLO, 10 mM MnCl₂, 150 mM NaCl, 20 mM HEPES pH 7.5, 0.035% DDM, 0.007% CHS and 20 µM of the acceptor peptide (for STT3A: 5CF-GSDANYTYTQ (SEQ ID No 33), for STT3B: 5CF-GSLANYTK (SEQ ID No 34)), and incubating at 30°C. 1 µL samples were taken at different time points and diluted in 10% ACN, 10 mM phosphate buffer pH 7. Samples were analyzed by reverse-phase chromatography using a UPLC Dionex UltiMate 3000 with an Accucore 150-C18 100 × 2.1 mm 2.6 µm column (Thermo Fisher Scientific)40. Peaks for glycopeptide and peptide were integrated using the Software ChromeleonTM, and the amount of produced glycopeptide was determined for each data point. Turnover rate determination was performed by fitting of the data to linear regression using PRISM software.

### Expression and purification of Trx-3(TbSTT3A-sequon) and Trx-3(TbSTT3B-sequon) constructs

Protein constructs to test protein glycosylation were expressed with a N-terminal *E. coli* thioredoxin tag and three glycosylation sequons. The thioredoxin N-terminus was followed by a linker to a His8 tag, another linker to a TEV cleavage sequence and a CGS linker to the peptide sequence containing the three glycosylation sequons. Two different constructs, Trx-3(TbSTT3A-sequon) (SEQ ID No 6) and Trx-3(TbSTT3B-sequon) (SEQ ID No 7) were designed based on the substrate preferences for TbSTT3A (GSDANYTYTQSEKSAASEANYTYSAEGRGSESDANYTYTK), SEQ ID No 4, and TbSTT3B (GSLANYTYTQSEKSDASLVNYTYSSEGRGSESLANYTYTEK), SEQ ID No 5. The glycosylation sequons for the constructs are underlined. The DNA sequences were codon optimized for expression in *Escherichia coli* (IDT web app) and were cloned into a modified pET vector using restriction free cloning.

Both constructs were expressed using *E. coli* BL21 (DE3) cells grown in ZYP-5052 autoinduction media at 37ºC for 4 hours followed by expression overnight at 16ºC. Cells were harvested by centrifugation, flash frozen in liquid nitrogen and stored at -80ºC until needed. Purification of both constructs was carried out with the same purification scheme at 4ºC. Cells were resuspended in lysis buffer (50 mM HEPES, pH 7.5; 500 mM NaCl; 10% glycerol; 1 mM TCEP) in a 1:5 weight ratio and phenylmethylsulfonyl fluoride and DNAsel (Roche) was added to final concentrations of 1 mM and 5 µg mL⁻¹, respectively. Cells were lysed with one pass in a microfluidizer (M-110P, Microfluidics) with a 200 µm ceramic cell equilibrated in lysis buffer and run at 15,000 psi. The lysate was then incubated at 60ºC for 30 minutes before cellular debris and aggerated protein was removed by centrifugation at 40,000 rpm in a Type 45Ti rotor (Beckman-Coulter) for 30 minutes. Imidazole, pH 7.5, was added to the supernatant to 20 mM before loading on a 5 mL Superflow Ni-NTA (Qiagen) column equilibrated in buffer A (20 mM HEPES, pH 7.5; 150 mM NaCl; 1 mM TCEP) with 20 mM imidazole added. Resin was washed with 50 mM imidazole in buffer A before protein elution in buffer A with 250 mM imidazole. Fractions containing the protein of interest were pooled and immediately desalted into buffer B (20 mM HEPES, pH 7.5; 1 mM TCEP, 0.5 mM EDTA). Protein was concentrated on a 10 kDa molecular weight cut off Amicon centrifugal filter (Merck Millipore) and flash frozen in liquid nitrogen.

### Fluorescein labelling of the Trx-3(TbSTT3A-sequon) and Trx-3(TbSTT3B-sequon) constructs

The cysteine residues of the Trx-3(TbSTT3A-sequon) construct (SEQ ID No 6) and the Trx-3(TbSTT3B-sequon) construct (SEQ ID No 7) were fluorescently labelled with fluorescein to facilitate detection on SDS-PAGE analysis using fluorescein-5-maleimide (Thermo Fisher). An aliquot of frozen protein was thawed, and a 100-fold molar excess of TCEP was added and incubated at room temperature for ~1 hour before desalting into reaction buffer (150 mM NaCl, 20 mM HEPES, pH 7.2). A 25-fold molar excess of fluorescein-5-maleimide dissolved in DMSO was added to the protein and incubated overnight at 4ºC in the dark. The reaction mixture was concentrated on a 10 kDa molecular weight cut off Amicon centrifugal filter (Merck Millipore) before SEC on a Superdex 200 increase column 10/300 (GE healthcare) at 0.5 mL min⁻¹ in reaction buffer. Protein fractions were pooled and flash frozen.

### In vitro protein glycosylation reactions

Protein glycosylation was carried out overnight at 30ºC with 5-10 µM fluorescein-Trx-3(TbSTT3A-sequon) or fluorescein-Trx-3(TbSTT3B-sequon), 0 to 40 µM LLO, and 250 nM TbSTT3A or TbSTT3B. The reactions were performed in 150 mM NaCl, 20 mM HEPES, pH 7.5, 10 mM MnCl₂, 0.035% DDM, and 0.007% CHS. Glycosylation of the fluorescein-Trx-3(TbSTT3A-sequon) construct was performed with TbSTT3A and Phy-PP-GlcNAc₂Man₅ LLO while the reactions with fluorescein-Trx-3(TbSTT3B-sequon) were performed with TbSTT3B and Phy-PP-GlcNAc₂Man₉. The samples were diluted in Laemmli buffer, separated using SDS-PAGE and visualized on a fluorescence scanner.

## Claims

1. A covalent conjugate between a monosaccharide and/or glycan and a lipid carrier molecule which is phytol.

2. The covalent conjugate of claim 1, wherein the glycan is a member selected from GIcNAc, GlcNAc₂, GlcNAc₂Man₁, GlcNAc₂Man₃, GlcNAc₂Man₅, GlcNAc₂Man₆, GlcNAc₂Man₇, GlcNAc₂Man₈, GlcNAc₂Man₉, GlcNAc₂Man₉Glc₁, GlcNAc₂Man₉Glc₂, GlcNAc₂Man₉Glc₃, or wherein the glycan comprises any of GIcNAc, GlcNAc₂, GlcNAc₂Man₁, GlcNAc₂Man₃, GlcNAc₂Man₅, GlcNAc₂Man₆, GlcNAc₂Man₇, GlcNAc₂Man₈, GlcNAc₂Man₉, GlcNAc₂Man₉Glc₁, GlcNAc₂Man₉Glc₂, GlcNAc₂Man₉Glc₃.

3. The covalent conjugate of claim 1, wherein the monosaccharide is mannose.

4. A method for forming the conjugate of any of claims 1 to 3 comprising contacting a monosaccharide or a glycan with the lipid carrier molecule phytol under conditions sufficient to covalently link the monosaccharide or the glycan to the lipid carrier molecule phytol.

5. A cell-free *in vitro* system for producing an N-glycan for glycosylating a peptide and/or polypeptide, said system comprising
- one or more covalent conjugates between a glycan and the lipid carrier molecule phytol,
- at least one of the enzyme SaAgIH (SEQ ID No 14, 15) of *Sulfolobus acidocaldarius* and/or the polypeptide CeALG2 (SEQ ID No 20, 21) of *Caenorhabditis elegans,* or an enzyme sharing at least 60%, 70%, 80%, 90%, 95%, 96%, 97%, 98%, or at least 99% sequence identity, or at least 70%, 80%, 90%, 95%, 96%, 97%, 98%, or at least 99% sequence similarity to one of said polypetides, and
- optionally, at least one of the ALG9 polypeptide of *Homo sapiens* HsALG9 (SEQ ID No 26, 27), the ALG12 polypeptide of *Gallus gallus* GgALG12 (SEQ ID No 28, 29), and/or the ALG3 polypeptide of *Saccharomyces cerevisiae* ScALG3 (SEQ ID No 24, 25), or an enzyme sharing at least 60%, 70%, 80%, 90%, 95%, 96%, 97%, 98%, or at least 99% sequence identity, or at least 70%, 80%, 90%, 95%, 96%, 97%, 98%, or at least 99% sequence similarity to any one of said polypeptides.

6. A cell-free *in vitro* system for producing a glycosylated peptide and/or polypeptide, said system comprising
- an oligosaccharyltransferase (OST) for transferring a glycan from a lipid carrier molecule to a glycosyl-moiety acceptor residue of a peptide or polypeptide,
- a peptide or polypeptide comprising one or more glycosyl-moiety acceptor residues, and
- one or more covalent conjugates between a glycan and the lipid carrier molecule phytol.

7. The system according to claim 6, wherein the glycosyl-moiety acceptor residue is an asparagine residue comprised in an *N*-linked glycosylation sequence of said peptide or polypeptide.

8. The system according to claim 7 wherein the OST is the eukaryotic single-subunit OSTTbSTT3A of *Trypanosoma brucei* (SEQ ID No 9) and/or the eukaryotic single-subunit OSTTbSTT3B of *T. brucei* (SEQ ID No 11), or an enzyme sharing at least 80%, 90%, 95%, 96%, 97%, 98%, or at least 99% sequence identity, or at least 85%, 90%, 95%, 96%, 97%, 98%, or at least 99% sequence similarity to one of said enzymes.

9. A cell-free *in vitro* method for forming of a glycosylated peptide or polypeptide, said method comprising contacting a peptide or polypeptide comprising an N-linked glycosylation sequence including an asparagine residue with a covalent conjugate between a glycan and the lipid carrier molecule phytol in the presence of an oligosaccharyltransferase (OST) under conditions sufficient for said OST to transfer the glycan from said covalent conjugate onto the asparagine of said *N*-linked glycosylation sequence, thereby forming a conjugate between said peptide or polypeptide and said glycan.

10. The system of any of claims 6 to 8, or the method of claim 9, wherein the *N*-linked glycosylation sequence is
LXNYT, (SEQ ID No 3),
wherein X is either alanine (A) or valine (V).

11. A peptide or polypeptide comprising an exogenous *N*-linked glycosylation sequence LXNYT, wherein X is either alanine (A) or valine (V).

12. Use of the peptide or polypeptide of claim 11 as a substrate for the single subunit OST TbSTT3B of *T. brucei* (SEQ ID No 11), or as a substrate for an enzyme sharing at least 80%, 90%, 95%, 96%, 97%, 98%, or at least 99% sequence identity, or at least 85%, 90%, 95%, 96%, 97%, 98%, or at least 99% sequence similarity to TbSTT3B (SEQ ID No 11).

13. An expression vector or a cell comprising a nucleic acid sequence encoding a peptide or polypeptide of claim 11.

14. A covalent conjugate between a peptide or polypeptide and a glycan, said peptide or polypeptide comprising one or more exogenous *N*-linked glycosylation sequence LXNYT (SEQ ID NO 3), wherein X is either alanine (A) or valine (V), wherein said glycan is covalently conjugated to said peptide or polypeptide at the asparagine (N) residue of said *N*-linked glycosylation sequence.

15. The covalent conjugate of claim 14, wherein the glycan is a member selected from GlcNAc₂, GlcNAc₂Man₁, GlcNAc₂Man₃, GlcNAc₂Man₅, GlcNAc₂Man₆, GlcNAc₂Man₇, GlcNAc₂Man₈, GlcNAc₂Man₉, GlcNAc₂Man₉Glc₁, GlcNAc₂Man₉Glc₂, GlcNAc₂Man₉Glc₃, or wherein the glycan comprises any of GlcNAc₂, GlcNAc₂Man₁, GlcNAc₂Man₃, GlcNAc₂Man₅, GlcNAc₂Man₆, GlcNAc₂Man₇, GlcNAc₂Man₈, GlcNAc₂Man₉, GlcNAc₂Man₉Glc₁, GlcNAc₂Man₉Glc₂, GlcNAc₂Man₉Glc₃.
